(19) Europäisches Patentamt
European Patent Office
Office européen des brevets

(11) Veröffentlichungsnummer: **0 037 504**
**B1**

(12) # EUROPÄISCHE PATENTSCHRIFT

(45) Veröffentlichungstag der Patentschrift: **23.02.83**

(51) Int. Cl.³: **C 07 C 149/12**, C 07 C 148/00, C 07 F 9/165

(21) Anmeldenummer: **81102162.5**

(22) Anmeldetag: **23.03.81**

(54) **Verfahren zur Herstellung von Dimethylpolysulfiden.**

(30) Priorität: **05.04.80 DE 3013396**

(43) Veröffentlichungstag der Anmeldung: **14.10.81 Patentblatt 81/41**

(45) Bekanntmachung des Hinweises auf die Patenterteilung: **23.02.83 Patentblatt 83/8**

(84) Benannte Vertragsstaaten: **BE CH DE FR GB IT LI NL**

(56) Entgegenhaltungen:
„Chemical Abstracts", Bd. 53, Nr. 22, 25. November 1959, Spalten 21 748-21 749, Columbus, Ohio, USA, G. Hilgetag *et al.*: „Thiophosphates. VI. Alkylating ester cleavage of dialkyl aryl and alkyl diaryl thiophosphates".
„Chemical Abstracts, Bd. 53, Nr. 21, 10. November 1959, Spalte 19 848c, Columbus, Ohio, USA, G. Hilgetag *et al.*: „Thiophosphates. II. The isomeric trimethyl thiophosphates as methylation agents".

(73) Patentinhaber: **BAYER AG, Zentralbereich Patente, Marken und Lizenzen, D-5090 Leverkusen 1, Bayerwerk (DE)**

(72) Erfinder: **Arold, Hermann, Dr., Falkenberg 151, D-5600 Wuppertal 1 (DE)**
Erfinder: **Humburger, Siegbert, Dr., Carl Rumpffstrasse 8, D-5090 Leverkusen (DE)**
Erfinder: **Diehr, Hans-Joachim, Dr., Höhe 35, D-5600 Wuppertal (DE)**
Erfinder: **Porkert, Helmut, Dr., c/o Mobay Chemical Coperation West Bay Road, Baytown, Tx 77520 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 037 504 B1

# Verfahren zur Herstellung von Dimethylpolysulfiden

Die vorliegende Erfindung betrifft ein neues technisch einfach durchführbares Verfahren zur Herstellung von bekannten Dimethylpolysulfiden durch Methylierung von Alkali- oder Erdalkalisulfiden mit O,O,O-Trimethylthiophosphorsäureester.

Bei technischen Herstellungsverfahren von O-Methylthiophosphorsäurechloriden, die als Zwischenstufen für Pflanzenschutzmittel verwendet werden, fällt O,O,O-Trimethylthiophosphorsäureester zwangsweise in grösseren Mengen an. Die Beseitigung dieser Verbindung kann ein ökologisches Problem darstellen und ist zudem mit hohen Kosten verbunden. Es besteht daher ein grosses Interesse O,O,O-Trimethylthiophosphorsäureester einer sinnvollen Verwendung zuzuführen.

Es wurde nun gefunden, dass man Dimethylpolysulfide der Formel I:

$$CH_3{-}S_x{-}CH_3 \qquad (I)$$

in welcher x eine ganze oder gebrochene Zahl zwischen 2 und 5, bedeutet, in hoher Ausbeute und guter Reinheit erhält, wenn man Polysulfide der Formel II:

$$M_yS_x \qquad (II)$$

in welcher M für ein Alkalimetallatom steht, wobei y die Zahl 2 bedeutet oder für ein Erdalkalimetallatom steht, wobei y die Zahl 1 bedeutet, wie Natrium, Kalium oder Calcium, vorzugsweise Natrium oder Kalium, insbesondere Natrium und x die oben angegebene Bedeutung hat, mit O,O,O-Trimethylthiophosphorsäureester der Formel III:

$$(CH_3O)_3P{=}S \qquad (III)$$

umsetzt.

Verwendet man z.B. Natriumpolysulfid der Formel $Na_2S_{3,5}$ und O,O,O-Trimethylthiophosphorsäureester, so kann der Reaktionsablauf durch das folgende Formelschema wiedergegeben werden:

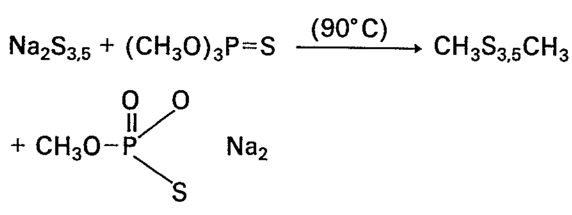


Der erfindungsgemäss als Ausgangsmaterial verwendbare O,O,O-Trimethylthiophosphorsäureester der Formel III ist eine bekannte Verbindung, ebenso wie die Polysulfide der Formel II, die (in Abhängigkeit des gewünschen Endproduktes) als Di-, Tri-, Tetra-, oder Pentasulfid oder Gemische dieser Sulfide eingesetzt werden können, wobei sich für einheitliche Verbindungen der Formel II (bzw. I) ganze Zahlen und bei Mischungen als Mittelwerte gebrochene Zahlen ergeben. In den Polysulfiden der Formel II steht M vorzugsweise für Natrium, oder Kalium, insbesondere für Natrium (y = 2).

Bei der Durchführung des erfindungsgemässen Verfahrens können die Molverhältnisse der Ausgangsverbindungen in einem weiten Bereich schwanken. Vorzugsweise setzt man 1 mol O,O,O-Trimethylthiophosphorsäureester der Formel III mit 0,5 bis 3 mol um, insbesondere mit 1 bis 2 mol Polysulfid der Formel II.

Die Umsetzung lässt man im allgemeinen bei Normaldruck ablaufen.

Die Umsetzung wird bevorzugt bei Temperaturen von 10 bis 200°C durchgeführt, insbesondere bei 20 bis 100°C und in einer besonderen ausführungsform der Erfindung bei Temperaturen zwischen 75 und 95°C.

Die Umsetzung kann in Wasser, in Gemischen von organischen Lösungsmitteln mit Wasser oder in organischen Lösungsmitteln durchgeführt werden, vorzugsweise in Wasser oder in Gegenwart von Wasser. Bei Mischungen von Wasser mit organischen Lösungsmitteln sind die Mischungsverhältnisse für das erfindungsgemässe Verfahren über weite Bereiche variabel. So können z.B. die Verhältnisse von Wasser zu organischem Lösungsmittel zwischen 1:100 und 100:1, vorzugsweise zwischen 1:10 und 10:1, liegen.

Als organische Lösungsmittel kommen vor allem polare Lösungsmittel in Frage, wie Alkohole, z.B. Methanol, Ethanol, n- und i-Propanol und n-Butanol, vorzugsweise Methanol, Ketone, z.B. Methylisobutylketon, cyclische Ether, z.B. Dioxan und Tetrahydrofuran und niedere Dialkylformamide z.B. Dimethylformamid. Bevorzugt sind organische polare Lösungsmittel, welche mit Wasser mischbar sind. Ganz besonders bevorzugt werden Wasser oder Methanol oder Mischungen von Wasser und Methanol in beliebigen Mischungsverhältnissen verwendet.

Die Aufarbeitung des Reaktionsgemisches erfolgt nach den allgemein üblichen Methoden. Wenn das erfindungsgemässe Verfahren in Wasser durchgeführt wird, können die entstandenen und abgeschiedenen Polysulfide der Formel I (welche in Wasser schwer löslich sind) nach den üblichen Methoden leicht abgetrennt werden. Wird als Lösungsmittel Wasser in Mischung mit einem organischen Lösungsmittel verwendet, muss nach Ablauf der Reaktion gegebenenfalls so viel Wasser hinzugefügt werden, dass das Polysulfid sich abscheidet. Analog ist zu verfahren, wenn ohne Wasserzusatz ein mit Wasser mischbares organisches Lösungsmittel eingesetzt wird. Falls ein nicht mit Wasser mischbares organisches Lösungsmittel verwendet wird, können durch Waschen des Reaktionsgemisches nach Ablauf der Reaktion die wasserlöslichen Nebenprodukte entfernt werden. Die Polysulfide der Formel I können in der organischen Lösung direkt weiter umgesetzt werden oder aus der Lösung nach üblichen Methoden isoliert werden (z.B. durch Abdestillieren des Lösungsmittels). Die erfindungsgemäss hergestellten Polysulfide sind von grosser Reinheit, so dass eine Reinigung (z.B. durch Destillation) für die

meisten Verwendungszwecke nicht erforderlich ist.

Die leicht wasserlöslichen, bei der Umsetzung entstehenden, Phosphorsäuremethylestersalze befinden sich bei der Verwendung von Wasser oder Mischungen von Wasser und organischen Lösungsmitteln als Lösungsmittel in der wässerigen Phase. Bei der Verwendung von nicht wässerigen Lösungsmitteln sind sie im Waschwasser enthalten, mit welchem in diesem Falle das Reaktionsgemisch nach der Umsetzung zweckmässigerweise gewaschen wird. Sie können in wässeriger Lösung oder direkt oder nach ihrer Isolierung z.B. als Zwischenprodukte für Pflanzenschutzmittel weiterverwendet werden. Falls eine solche Verwendung nicht gewünscht wird, können sie wesentlich einfacher als Abfallstoffe beseitigt werden als der eingesetzte Trimethylthiophosphorsäureester.

Das erfindungsgemässe Verfahren kann nach einer besonderen Ausführungsform auch mehrstufig in Kaskadenschaltung oder kontinuierlich in üblicher Weise durchgeführt werden.

Die nach dem erfindungsgemässen Verfahren herstellbaren Dimethylpolysulfide fallen in einer sehr hohen Reinheit an. Sie sind wertvolle Zwischenprodukte z.B. zur Herstellung von bekannten Methylthiophenolen, die ihrerseits z.B. als Zwischenprodukte für Pflanzenschutzmittel, verwendet werden.

Die Durchführung des erfindungsgemässen Verfahrens soll anhand der folgenden Beispiele erläutert werden:

*Beispiel 1*

Zu 943 g einer 45%igen (Gewichtsprozente) Lösung von Natriumpolysulfid der Zusammensetzung $Na_2S_{3,5}$ (2,6 mol) gibt man innerhalb 2 h unter Rühren bei 85 bis 90°C 312 g (2 mol) O,O,O-Trimethylthiophosphorsäureester. Nach der Zugabe wird die Reaktionsmischung noch 5 h bei 90°C gerührt. Anschliessend wird der Ansatz auf 20°C abgekühlt und 280 g Dimethylpolysulfid (1,97 mol) als organische Phase abgetrennt.

Weitere Beispiele enthält die nachfolgende Tabelle unter Angabe der wesentlichen Reaktionsbedingungen.

Tabelle

| Beispiel | O,O,O-Trimethylthiophosphorsäureester (g) | Polysulfid | Lösungsmittel | Reaktionszeit (h) | Reaktionstemperatur (°C) | Dimethylpolysulfid (g) |
|---|---|---|---|---|---|---|
| 2 | 156 | 99 g $Na_2S_{3,5}$ | $H_2O$/Methanol (3:1)* | 12 | 25 | 70 |
| 3 | 156 | 99 g $Na_2S_{3,5}$ | $H_2O$/Methanol (3:1)* | 8 | 50 | 72 |
| 4 | 156 | 197,5 g $Na_2S_{3,5}$ | $H_2O$ | 8 | 60 | 135 |
| 5 | 156 | 197,5 g $Na_2S_{3,5}$ | $H_2O$ | 7 | 90 | 140 |
| 6 | 156 | 202,5 g $CaS_{3,5}$ | $H_2O$ | 8 | 90 | 138 |
| 7 | 156 | 197,5 g $Na_2S_{3,5}$ | Methanol | 8 | 60 | 135 |

* Volumenteile.

## Patentansprüche

1. Verfahren zur Herstellung von Dimethylpolysulfiden der Formel (I):

$$CH_3-S_x-CH_3 \qquad (I)$$

in welcher x eine Zahl zwischen 2 und 5 bedeutet, dadurch gekennzeichnet, dass man Polysulfide der Formel (II):

$$M_yS_x \qquad (II)$$

in welcher M für ein Alkalimetallatom steht, wobei y die Zahl 2 bedeutet oder für ein Erdalkalimetallatom steht, wobei y die Zahl 1 bedeutet und x die oben angegebene Bedeutung hat, mit O,O,O-Trimethylthiophosphorsäureester der Formel (III):

$$(CH_3O)_3P{=}S \qquad (III)$$

umsetzt, und die entstandenen Polysulfide der Formel (I) isoliert, und gegebenenfalls reinigt, und gewünschtenfalls die ebenfalls gebildeten Phosphorsäuremethylestersalze zu weiterer Verwendung gewinnt.

2. Verfahren nach Anspruch 1, dadurch gekennzeichnet, dass man die Umsetzung bei Temperaturen zwischen 10 und 200°C vornimmt.

3. Verfahren nach Anspruch 1, dadurch gekennzeichnet, dass man die Umsetzung bei Temperaturen zwischen 20 und 100°C vornimmt.

4. Verfahren nach Anspruch 1, dadurch gekennzeichnet, dass man die Umsetzung bei Temperaturen zwischen 75 und 95°C vornimmt.

5. Verfahren nach den Ansprüchen 1 bis 4, dadurch gekennzeichnet, dass in Formel (II) M für Natrium und y für 2 steht.

6. Verfahren nach den Ansprüchen 1 bis 5, dadurch gekennzeichnet, dass man als Lösungsmittel Wasser verwendet.

7. Verfahren nach den Ansprüchen 1 bis 5, dadurch gekennzeichnet, dass man als Lösungsmittel Methanol verwendet.

8. Verfahren nach den Ansprüchen 1 bis 5, dadurch gekennzeichnet, dass man als Lösungsmittel Wasser/Methanol-Mischungen verwendet.

## Claims

1. Process for the preparation of dimethyl polysulphides of the formula (I):

$$CH_3-S_x-CH_3 \quad (I)$$

in which x denotes a number between 2 and 5, characterized in that polysulphides of the formula (II):

$$M_yS_x \quad (II)$$

in which M represents an alkali metal atom, in which case y represents the number 2, or represents an alkaline earth metal atom, in which case y denotes the number 1, and x has the meaning indicated above, are reacted with O,O,O-trimethyl thiophosphates of the formula (III):

$$(CH_3O)_3P{=}S \quad (III)$$

and the polysulphides of the formed formula (I) are isolated and, if appropriate, purified and, if desired, the phosphoric acid methyl ester salts also formed are isolated for further use.

2. Process according to claim 1, characterized in that the reaction is carried out at temperatures between 10 and 200°C.

3. Process according to claim 1, characterized in that the reaction is carried out at temperatures between 20 and 100°C.

4. Process according to claim 1, characterized in that the reaction is carried out at temperatures between 75 and 95°C.

5. Process according to claims 1 to 4, characterized in that in formula (II) M represents sodium and y represents 2.

6. Process according to claims 1 to 5, characterized in that water is used as the solvent.

7. Process according to claims 1 to 5, characterized in that methanol is used as the solvent.

8. Process according to claims 1 to 5, characterized in that mixtures water/methanol are used as the solvent.

## Revendications

1. Procédé de préparation de polysulfures de diméthyle de formule (I):

$$CH_3-S_x-CH_3 \quad (I)$$

dans laquelle x est un nombre dont la valeur va de 2 à 5, caractérisé en ce que l'on fait réagir des polysulfures de formule (II):

$$M_yS_x \quad (II)$$

dans laquelle M représente un atome de métal alcalin et y est alors égal à 2, ou représente un atome de métal alcalino-terreux, auquel cas y est égal à 1 et x a la signification indiquée ci-dessus, avec le thiophosphate d'O,O,O-triméthyl de formule (III):

$$(CH_3O)_3P{=}S \quad (III)$$

on isole les polysulfures de formule (I) ainsi obtenus et, le cas échéant, on les purifie et, si on le désire, on isole des sels de phosphates de méthyle également formés pour une autre utilisation.

2. Procédé selon la revendication 1, caractérisé en ce que l'on effectue la réaction à des températures allant de 10 à 200°C.

3. Procédé selon la revendication 1, caractérisé en ce que l'on effectue la réaction à des températures allant de 20 à 100°C.

4. Procédé selon la revendication 1, caractérisé en ce que l'on effectue la réaction à des températures de 75 à 95°C.

5. Procédé selon les revendications 1 à 4, caractérisé en ce que, dans la formule (II), M représente le sodium et y est égal à 2.

6. Procédé selon les revendications 1 à 5, caractérisé en ce que l'on utilise l'eau en tant que solvant.

7. Procédé selon les revendications 1 à 5, caractérisé en ce que l'on utilise le méthanol en tant que solvant.

8. Procédé selon les revendications 1 à 5, caractérisé en ce que l'on utilise des mélanges eau/méthanol en tant que solvants.